# EUROPEAN PATENT APPLICATION

(11) **EP 4 502 073 A1**
(43) Date of publication of application: **05.02.2025**
(21) Application number: 23775116.9
(22) Date of filing: 24.03.2023
(51) Int. Cl.: C08L 101/00, B29B 13/08, B29C 45/00, B29C 45/72, C08K 5/3445

(54) **MICROWAVE ABSORBER**

(30) Priority: 24.03.2022 JP 2022049052
(71) Applicant: Microwave Chemical Co., Ltd., Osaka-shi, Osaka 559-0025 (JP); OSAKA UNIVERSITY, Suita-shi Osaka 565-0871 (JP)
(72) Inventor: WADA, Naoyuki, Osaka-shi, Osaka 559-0025 (JP); MORIKAWA, Maki, Osaka-shi, Osaka 559-0025 (JP); KAIHARA, Kanako, Osaka-shi, Osaka 559-0025 (JP); TSUKAHARA, Yasunori, Osaka-shi, Osaka 559-0025 (JP); YASUDA, Makoto, Suita-shi, Osaka 565-0871 (JP); NISHIMOTO, Yoshihiro, Suita-shi, Osaka 565-0871 (JP)
(74) Representative: Müller-Boré & Partner Patentanwälte PartG mbB
(86) International application number: PCT/JP2023/012034
(87) International publication number: WO 2023/182523

(57) **Abstract**

The present invention provides a novel microwave absorber that does not exhibit black color and is solid at room temperature. A microwave absorber including an imidazolium salt represented by a formula (1) (in the formula, R¹ represents a linear alkyl group having the number of carbon atoms equal to or less than the number of carbon atoms of the alkyl group of R², R² represents an alkyl group having 9 or more carbon atoms, R³ and R⁴ may be the same or different from each other and represent hydrogen or an alkyl group having 1 to 6 carbon atoms, and X represents a halogen element) is useful as a novel microwave absorber that does not exhibit black color and is solid at room temperature.

## Description

### Technical Field

The present invention relates to a microwave absorber. More specifically, the present invention relates to a microwave absorber that is suitable for microwave melting of resins, and that does not remain as a black substance in resin pellets or resin molded bodies, and does not remain as a liquid.

### Background art

The molding by mold, in which molten resin is poured into a mold and solidified to be molded, is one of the most widely used resin molding techniques.

In the molding by mold, in order to mold a desired shape, the fluidity of the molten resin needs to be made sufficiently high so that the molten resin can reach the fine portions of the mold. As a means of heat molding the resin by the molding by mold, a means of transferring heat from the outside of the mold is generally used.

On the other hand, as a means of heating various materials instead of heat transfer, microwave heating may be used, and methods for performing microwave heating more efficiently are being researched.

For example, PTL 1 discloses a microwave-heatable heat storage material, which includes a combination of additives made of: a solid molten mixture of about 84 to 92% by weight of a phase change material such as a polyolefin resin and an ethylene copolymer, in order to allow rapid heating in a microwave oven without reducing the heat storage capacity; and about 8 to 16% by weight of fine silica particles and about 0.5 to 15% by weight of conductive carbon black, and describes that the heat storage material is molded into the form of pellets, thin plates, moldings, or the like of the solid molten mixture.

PTL 2 discloses a conductive resin composition for microwave heating, including: a non-carbonaceous conductive filler, a curable insulating binder resin, and a carbonaceous material having a higher volume resistivity than the non-carbonaceous conductive filler, as a conductive resin composition for microwave heating that can exert high conductivity when cured, and can be heated and cured uniformly in a short time while suppressing the generation of sparks when heated by microwaves; and including 1 to 20 parts by mass of graphite particles having an aspect ratio of 20 or less with respect to the total of 100 parts by mass of the non-carbonaceous conductive filler and the curable insulating binder resin.

NPLs 1 and 2 describe that using microwave heating in the heat molding of carbon fiber reinforced plastic (CFRP) causes the carbon fibers in the CFRP to selectively absorb the microwaves, allowing rapid heating from the inside.

On the other hand, in the field of organic synthesis, ionic liquids have been reported as novel, recyclable and environmentally friendly materials to replace dipolar aprotic solvents for organic synthesis. Ionic liquids are known to absorb microwave radiation extremely efficiently, be readily soluble in a variety of organic solvents, and be able to be used to increase microwave absorption in low-absorbing reaction mixtures (NPL 3).

### Citation List

### Patent Literature

PTL1: Japanese Patent Application Laid-Open No. H10-067981
PTL 2: WO 2014/196444 A

### Non Patent Literature

NPL 1: Compos. Sci. Technol., 68 (2008) 1854-1861
NPL 2: Adv. Compos. Mater., 25 (2016) 71-79
NPL 3: "NL8 INI Tech Tips_solvent", [online], October 29, 2009, Biotage Japan Ltd., [searched on December 24, 2021], Internet <URL: https://www.biotage.co.jp/archive_newsletter/nl8_initech/>

### Summary of Invention

### Technical Problem

When a resin is heated by microwaves, as described above, there is used a means of coexisting carbon black, graphite particles, carbon fibers, or the like, which has high microwave absorption capacity, with the resin to be heated as a microwave absorbing material. However, in resin products produced by such means, the resin solidifies or cures together with the material used as the microwave absorbent, and thus the black color of the material itself inevitably appears on the surface.

On the other hand, as described above, ionic liquids used as solvents for organic chemical synthesis have high microwave absorption capabilities, and thus it is possible to produce resin products that do not exhibit a black color by using ionic liquids as microwave absorbers instead of the above-described materials used for resin heating. However, ionic liquids are liquid at room temperature, and thus remain as liquid even after the resin has solidified or cured, which may impair the properties or mechanical characteristics of the resin product.

Therefore, an object of the present invention is to provide a novel microwave absorber that is not exhibit black color and is solid at room temperature.

### Solution to Problem

As a result of extensive investigations, the present inventors have found that an imidazolium salt having a specific structure can achieve the above-described object of the present invention, and have thus completed the present invention.

That is, the present invention provides the following aspects.
Clause 1. A microwave absorber including an imidazolium salt represented by a formula (1): 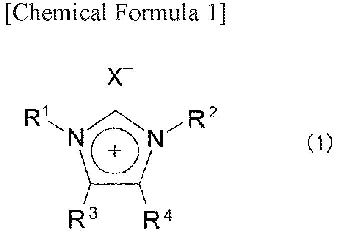 where R¹ represents a linear alkyl group having the number of carbon atoms equal to or less than the number of carbon atoms of an alkyl group of R², R² represents an alkyl group having 9 or more carbon atoms, R³ and R⁴ represent hydrogen or an alkyl group having 1 to 6 carbon atoms that may be the same or different from each other, and X represents a halogen element.
Clause 2. The microwave absorber according to clause 1, in which the number of carbon atoms of the alkyl group of R² is 9 to 22.
Clause 3. The microwave absorber according to clause 1 or 2, in which the halogen element is chlorine or bromine.
Clause 4. The microwave absorber according to any one of clauses 1 to 3, in which a difference between the number of carbon atoms of R¹ and the number of carbon atoms of R² is 10 to 12, and the halogen element is chlorine.
Clause 5. The microwave absorber according to any one of clauses 1 to 4, which is used by being mixed with a resin to be microwave-heated.
Clause 6. A resin composition including the microwave absorber according to any one of clauses 1 to 5 and a resin.
Clause 7. The resin composition according to clause 6, in which the resin is a thermoplastic resin and has a pellet shape.
Clause 8. A method for heating a resin using the microwave absorber according to any one of clauses 1 to 5.
Clause 9. The method according to clause 8, in which the microwave absorber is used at a ratio of 0.01 to 10 parts by weight to 100 parts by weight of the resin.
Clause 10. A method for producing a resin molded body, the method including: a step 1 of heating the resin composition according to clause 6 or 7 with a microwave to provide a flowable resin composition; and a step 2 of molding the flowable resin composition.
Clause 11. The method according to clause 10, further including a step of heating the flowable resin composition with a microwave to maintain flowability.
Clause 12. The method according to clause 10 or 11, in which the step 2 is injection molding.

### Advantageous Effects of Invention

According to one aspect of the present invention, there is provided a microwave absorber that does not remain as a black substance nor remains as a liquid after solidification or curing of a resin, and a method for producing a resin product using the microwave absorber.

### Description of Embodiments

### [1. Microwave absorber]

The microwave absorbing material means a material that absorbs microwaves and generates heat. The microwave absorber of the present invention includes an imidazolium salt represented by a formula (1).

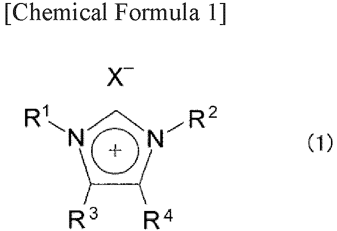

In the formula (1), R¹ represents a linear alkyl group having the number of carbon atoms equal to or less than the number of carbon atoms of the alkyl group of R². The number of carbon atoms in the linear alkyl group of R¹ is not particularly limited as long as it is equal to or less than the number of carbon atoms in the alkyl group of R², but it is preferably 1 to 14, more preferably 1 to 8, still more preferably 1 to 6, and particularly preferably 1 to 2 from the viewpoint of improving the melting efficiency of the resin by microwave heating or, in addition to this viewpoint, from the viewpoint of suppressing discoloration of the molded body.

In the formula (1), R² represents an alkyl group having 9 or more carbon atoms. The alkyl group of R² may be any of a linear alkyl group, a branched alkyl group, an alicyclic alkyl group, and the like. From the viewpoint of improving the melting efficiency of the resin by microwave heating or, in addition to this viewpoint, from the viewpoint of suppressing discoloration of the molded body, the number of carbon atoms of the alkyl group of R² is preferably 9 to 22, and the lower limit value of the range (9 to 22) may be 11 or more or 13 or more, and the upper limit value of the range (9 to 22) may be 14 or less. Among these examples of R², a linear alkyl group having 9 to 22 carbon atoms and more preferably 11 to 14 carbon atoms is particularly exemplified from the viewpoint of improving the melting efficiency of the resin by microwave heating or, in addition to this viewpoint, from the viewpoint of suppressing discoloration of the molded body.

In the formula (1), R³ and R⁴ represent hydrogen or an alkyl group having 1 to 6 carbon atoms, which may be the same or different from each other. The alkyl group having 1 to 6 carbon atoms is preferably a linear alkyl group, more preferably an alkyl group having 1 to 4 carbon atoms, still more preferably an alkyl group having 1 to 3 carbon atoms, and still more preferably an alkyl group having 1 or 2 carbon atoms. From the viewpoint of improving the melting efficiency of the resin by microwave heating or, in addition to this viewpoint, from the viewpoint of suppressing discoloration of the molded body, R³ and R⁴ are preferably hydrogen.

In the formula (1), X represents a halogen element. Examples of the halogen element include chlorine, bromine, and iodine, and chlorine and bromine are preferable from the viewpoint of suppressing coloring of the resin pellet when kneaded into the resin pellet and/or from the viewpoint of suppressing discoloration of the molded body.

Among the compounds represented by the formula (1), a compound in which the difference between the carbon number of R¹ and the carbon number of R² is 10 to 12, R³ and R⁴ are hydrogens, and the halogen element is chlorine is particularly preferable from the viewpoint of improving the melting efficiency of the resin by microwave heating or, in addition to this viewpoint, from the viewpoint of suppressing discoloration of the molded body.

The microwave absorber of the present invention can be used by being mixed with a resin to be microwave-heated in order to heat the resin with microwaves. The resin generally does not absorb microwaves, and thus the resin can be efficiently heated by mixing the microwave absorber of the present invention in the resin and causing the microwave absorber to generate heat by microwave irradiation.

### [2. Resin composition]

In order to heat a resin using the microwave absorber according to "1. Microwave absorber", the microwave absorber is mixed previously with a resin to be microwave-heated. Therefore, the present invention also provides a resin composition containing the microwave absorber and a resin.

The resin composition of the present invention is formed on the premise of being processed by microwave heating. The resin may be either a thermoplastic resin or a thermosetting resin.

Examples of the thermoplastic resin contained in the resin composition of the present invention include: crystalline resins of polyolefins (PO) such as polyethylene (PE) and polypropylene (PP); polyamide (PA) such as nylon; polyesters such as polyethylene terephthalate (PET) and polybutylene terephthalate (PBT); polyacetal (POM); polyetheretherketone (PEEK); and fluororesins such as polytetrafluoroethylene (PTFE). In addition, examples of the thermoplastic resin include: amorphous resins of polyvinyl chloride (PVC); styrene resins such as polystyrene (PS), styrene acrylonitrile resin (SAN), and acrylonitrile butadiene styrene resin (ABS); acrylic resin such as polymethyl methacrylate (PMMA); and polycarbonate (PC). These thermoplastic resins may be used singly or in combination of two or more types thereof.

The thermosetting resin contained in the resin composition of the present invention is a thermoplastic resin that is not completely cured, that is, an uncured thermoplastic resin or a semi-cured thermoplastic resin. Examples of the thermosetting resin include phenol resin (PF), epoxy resin (EP), melamine resin (MF), urea resin (UF), unsaturated polyester resin (UP), alkyd resin, silicone resin, polyurethane (PUR), and thermosetting polyimide (PI). These thermosetting resins may be used singly or in combination of two or more types thereof.

In the present invention, a person skilled in the art can appropriately select an appropriate resin from the above resins according to the use of the resin product to be produced. Among the above resins, thermoplastic resins are preferable, crystalline resins are more preferable, and polyolefins are more preferable.

As the microwave absorber contained in the resin composition of the present invention, one type of the microwave absorbers described in "1. Microwave absorber" may be used singly, or two or more types thereof may be used in combination.

If the resin composition of the present invention is a resin composition containing a microwave absorber and a thermoplastic resin, specific examples of the shape of the resin composition include a pellet shape. Specific examples of the pellet-shaped resin composition include natural pellets (uncolored pellets), masterbatches, and colored pellets.

If the resin composition of the present invention is a resin composition containing a microwave absorber and a thermosetting resin, specific forms of the resin composition include a thermosetting resin composition. Further, the thermosetting resin composition may be in the form of a prepreg. The prepreg is obtained by impregnating fibers with a resin composition containing a microwave absorber and a thermosetting resin. The microwave absorber contained in the resin composition of the present invention does not exhibit a black color as in the carbon compound, and thus fibers that do not exhibit a black color as such, specifically, aramid fibers, glass fibers, and the like are preferably used as the fibers constituting the prepreg.

The resin composition of the present invention may further contain not only the above components but also other additives. Examples of other additives include a pigment, a dye, a resin stabilizer, a stabilization aid, an antioxidant, an ultraviolet absorber, a radical scavenger, a lubricant, an antistatic agent, and a flame retardant, and one or more of types thereof can be appropriately used. It is preferable that these other additives do not exhibit black color.

### [3. Method for heating resin]

The microwave absorber described in "1. Microwave absorber" is typically used for heating a resin. Therefore, the present invention also provides a method for heating a resin using the microwave absorber.

The method for heating the resin of the present invention typically includes a step of heating the resin composition according to [2. Resin composition] by irradiating with a microwave.

In the method for heating the resin of the present invention, the resin to be heated is as described in [2. Resin composition].

The amount of the microwave absorber used can be appropriately set according to the type of the microwave absorber. For example, the amount of the microwave absorber used may be 0.01 to 10 parts by weight, 0.01 to 5 parts by weight, 0.01 to 3 parts by weight, or 0.01 to 1.5 parts by weight with respect to 100 parts by weight of the resin.

More specifically, in the microwave absorber, if the halogen element in the formula (1) is chlorine, the amount of the microwave absorber used is, from the viewpoint of saving the microwave output power for maintaining the heated state of the resin and/or from the viewpoint of suppressing alteration of the resin after solidification or curing, a proportion of 0.04 to 10 parts by weight, preferably 0.09 to 10 parts by weight, more preferably 0.9 to 10 parts by weight, 0.9 to 5 parts by weight, 0.9 to 3 parts by weight, or 0.9 to 1.5 parts by weight with respect to 100 parts by weight of the resin.

In addition, in the microwave absorber, if the halogen element in the formula (1) is bromine or iodine, the amount of the microwave absorber used is, from the viewpoint of saving the microwave output power for maintaining the heated state of the resin and/or from the viewpoint of suppressing alteration of the resin after solidification or curing, a proportion of 0.09 to 10 parts by weight, preferably 0.9 to 10 parts by weight, 0.9 to 5 parts by weight, 0.9 to 3 parts by weight, or 0.9 to 1.5 parts by weight with respect to 100 parts by weight of the resin.

Further, in the microwave absorber, if the difference between the number of carbon atoms of R¹ and the number of carbon atoms of R² is 10 to 12 in the formula (1), and the halogen element is chlorine, the amount of the microwave absorber used is, from the viewpoint of saving the microwave output power for maintaining the heated state of the resin and/or from the viewpoint of suppressing alteration of the resin after solidification or curing, a proportion of 0.01 to 10 parts by weight, preferably 0.04 to 10 parts by weight, more preferably 0.09 to 10 parts by weight, still more preferably 0.9 to 10 parts by weight, 0.9 to 5 parts by weight, 0.9 to 3 parts by weight, or 0.9 to 1.5 parts by weight with respect to 100 parts by weight of the resin.

As the heating temperature (temperature of the heated resin), a temperature at which the resin becomes flowable can be appropriately set according to the type of the resin. Specifically, if a crystalline resin is used as the resin, in order to melt the crystalline resin, in a case where the melting point of the crystalline resin is set to T1 (°C), the temperature is allowed to reach T1 or more and T2 or less (with the proviso that T2 (°C) is from T1+10°C to T1+50°C) (temperature for melting), and thereafter, in order to maintain the molten state, a temperature at which the molten state can be maintained (preferably, the temperature for the melting) can be maintained. If an amorphous resin is used as the resin, in order to melt the amorphous resin, in a case where the glass transition point of the amorphous resin is set to t1 (°C), the temperature is allowed to reach t1 or more and t2 or less (with the proviso that t2 (°C) is from 11 + 10°C to t1+50°C) (temperature for melting), and thereafter, in order to maintain the molten state, a temperature at which the molten state can be maintained (preferably, the temperature for the melting) can be maintained. If a thermosetting resin is used as the resin, in order to flow the thermosetting resin, the temperature is allowed to reach a temperature that enables to flow the thermosetting resin, and then is allowed to reach a curing temperature of the thermosetting resin.

The condition of the microwave can be appropriately set according to the temperature condition.

The frequency of the microwave is not particularly limited, and is, for example, 300 MHz to 300 GHz, preferably 900 MHz to 36 GHz, more preferably 1 to 24 GHz, and more preferably 2.4 to 10 GHz. The microwave may be an electromagnetic wave having a wavelength of 1 mm to 1 m.

The output power of the microwave is not particularly limited, and can be appropriately set according to the amount of resin to be heated and the temperature to be reached and maintained. Specifically, the output power for flowing the resin is, for example, 10 W to 10 kW.

### [4. Method for producing resin molded body]

The present invention further provides a method for producing a resin molded body. The method for producing a resin molded body of the present invention includes: a step 1 of heating the resin composition described in "2. Resin composition" with a microwave to provide a flowable resin composition; and a step 2 of molding the flowable resin composition. The method for producing a resin molded body of the present invention may further include a step of heating the flowable resin composition with a microwave to maintain flowability.

Details of the resin composition used in the step 1 are as described in "2. Resin composition". In addition, the heating conditions in the step 1 and the optional step of maintaining flowability are as described in [3. Method for heating resin].

The specific method of the molding method in the step 2 is not particularly limited, and any method capable of molding the flowable resin composition can be used. Examples of the molding method include injection molding, extrusion molding, blow molding, and vacuum casting. Among these molding methods, injection molding is preferable. The material of the molding mold is not limited to a metal (in this case, the molding mold is a so-called metal mold) and includes a non-metal, and examples of the non-metal include a resin (preferably silicone) and an inorganic substance.

If the production method of the present invention further includes a step of maintaining the above-described flowability, a specific form of the step may be, for example, a form in which the flowable resin composition injected or extruded from an injector or extruder, and then introduced into a molding mold is heated by microwaves. This step maintains the flowability of the flowable resin composition without cooling inside the mold, and thus can be used, for example, to allow the resin composition to permeate fine portions inside the mold, thereby producing a resin molded product with high molding precision.

### Examples

The present invention will be described in more detail below with reference to examples, but the present invention is not limited to the following examples.

### [Test Example 1]

A compound having the structure shown in Table 1 (R¹, R², and X of the compound shown in the formula (1) are specifically shown) were prepared as a microwave absorber. The melting points, properties at 25°C, and colors at 25°C of these microwave absorber are shown in Table 1.

One part by weight of a microwave absorber was added to 100 parts by weight of polypropylene resin, and was melt-heated at 200°C in a nitrogen atmosphere and mixed, and then cooled to prepare microwave absorber-containing resin pellets (hereinafter referred to as "resin pellets"). The properties of the obtained resin pellets at 25°C were confirmed, and further, for those that were solid in properties, the color at 25°C was also evaluated.

0.5 g of resin pellets, which are solid at 25°C, were placed in a quartz tube with an inner diameter of 8 mm and a thickness of 1 mm, and were heated to 200°C (that is, the temperature for melting) by irradiating with microwaves of 2450 MHz using a microwave reaction device MR-2G-100 manufactured by Ryowa Electronics Co., Ltd. and melted. For melting, initial heating was performed at 20 W, and if no temperature increase was observed within 30 seconds of starting microwave irradiation at the specified output power, the output power was increased by 10 W at a time until a temperature of 200°C for melting was reached. Thereafter, the molten state was maintained by maintaining the temperature at 200°C for 2 minutes. Table 1 shows the output power (W) required to bring the resin pellets to 200°C, which is the temperature for melting, and the output power (W) required to maintain the molten state of the molten resin pellets. As the output power is lower, the microwave absorption capacity is evaluated to be higher.

After remaining in the molten state for 2 minutes, the resin was cooled to room temperature and allowed to be solidified. This resulted in a model of the injection molded body. The color of the resin pellets was used as the standard, and the effect of suppressing color change of the molded body (model) was evaluated based on the following criteria. If the color change inhibition is rated as ⊚ or ∘, it can be evaluated that the color change inhibition is sufficient in an extent of not impairing the general-purpose use in the production of resin molded bodies with high brightness. The results are shown in Table 1.
⊚: Almost no color change is observed
○: The color change is slight (if the resin pellets are white or light yellow, they will turn light brown, and if the resin pellets are yellow, they will turn brown)
△: The color change is more significant than the above ∘

**[Table 1]**

| | Microwave absorber | | 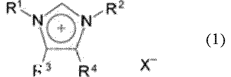 | | | (1) | Resin pellet | | Microwave absorption capacity (required output power) | | Molded body |
|---|---|---|---|---|---|---|---|---|---|---|---|
| | R¹ | R² | X⁻ | Melting point | Property at 25°C | Color at 25°C | Property at 25°C | Color at 25°C | Molten | To maintain molten state | Suppression of color change |
| Example 1 | Me | C₁₁H₂₃ | Cl⁻ | 53°C | Solid | White | Solid | White | 40 W | 29 W | ⊚ |
| Example 2 | Me | C₁₂H₂₅ | Cl⁻ | 53°C | Solid | White | Solid | White | 30 W | 23 W | ⊚ |
| Example 3 | Me | C₁₃H₂₇ | Cl⁻ | 61°C | Solid | White | Solid | White | 30 W | 21 W | ○ |
| Example 4 | Me | C₁₄H₂₉ | Cl⁻ | 64°C | Solid | White | Solid | White | 50 W | (14 W) | Δ |
| Example 5 | Me | C₁₄H₂₉ | Br ⁻ | 54°C | Solid | White | Solid | White | 40 W | 24 W | ⊚ |
| Example 6 | Me | C₁₂H₂₅ | I⁻ | / | Solid | Orange | Solid | Yellow | 30 W | 27 W | Δ |
| Example 7 | Et | C₁₄H₂₉ | Cl⁻ | 50°C | Solid | White | Solid | White | 40 W | 19 W | ○ |
| Example 8 | C₁₄H₂₉ | C₁₄H₂₉ | Cl⁻ | 63°C | Solid | White | Solid | White | 70 W | (33 W) | Δ |
| Example 9 | C₈H₁₇ | C₁₄H₂₉ | Cl⁻ | 52°C | Solid | Yellow | Solid | Light yellow | 80 W | 41 W | ○ |
| Comparative Example 1 | 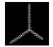 | C₁₄H₂₉ | Cl⁻ | < 25°C | Liquid | Light yellow | Solid/ liquid | | | | |
| Comparative Example 2 | Me | Et | Cl⁻ | < 25°C | Liquid | Yellow | Solid/ liquid | | | | |
| Comparative Example 3 | Bu | Bu | Cl⁻ | < 25°C | Liquid | Light yellow | Solid/ liquid | | | | |
| Comparative Example 4 | Me | 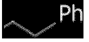 | Cl⁻ | | Solid/ liquid | Light orange | Solid/ liquid | | | | |
| Comparative Example 5 | Me | 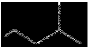 | Cl⁻ | < 25°C | Liquid | Light orange | Solid/ liquid | | | | |
| Comparative Example 6 | Me | 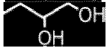 | Cl⁻ | < 25°C | Liquid | Orange | Solid/ liquid | | | | |

| | | | | | | | | | | | |
|---|---|---|---|---|---|---|---|---|---|---|---|
| * In all of Examples and Comparative Examples, R³ and R⁴ represent H. | | | | | | | | | | | |

As is clear from the comparison between Examples 1 to 9 and Comparative Examples 1 to 6, the specific compounds used in Examples 1 to 9 (that is, compounds defined in the formula (1), in which R¹ is a linear alkyl group having the number of carbon atoms equal to or less than the number of carbon atoms of the alkyl group of R², R² is an alkyl group having a carbon number of 9 or more, and X is a halogen element) were solid at room temperature, and were also solid when kneaded into a resin to form resin pellets. Further, the specified compound effectively functions as a microwave absorber in resin molding, and a molded body having solid properties was able to be obtained. Among these, the compounds used in Examples 1, 2, 3, 5, 6, and 7 were excellent in microwave absorption efficiency, and the compounds used in Examples 2, 3, and 6 were even more excellent in microwave absorption efficiency. Some of the molded bodies obtained in Examples 1 to 9 showed slight discoloration, but no black discoloration was observed, which is observed when carbon black, graphite particles, carbon fibers, and the like are used as microwave absorbers.

### [Test Example 2]

The same operation as in Test Example 1 was performed, except that when the compound shown in Table 2 was used as the microwave absorber, the amount of the microwave absorber used with respect to 100 parts by weight of the polypropylene resin was changed to the amount shown in Table 2. Table 2 shows the output power (W) required to maintain the molten state of the molten resin pellets.

**[Table 2]**

| | Microwave absorber | | | Amount added (parts by weight) | Output power required to maintain molten state |
|---|---|---|---|---|---|
| | R1 | R2 | X⁻ | | |
| Example 10 | Me | C₁₃H₂₇ | Cl⁻ | 0.01 | 100 W |
| Example 11 | | | | 0.05 | 80 W |
| Example 12 | | | | 0.1 | 54 W |
| Example 3 | | | | 1 | 21 W |
| Example 13 | | | | 10 | 10 W |
| Example 14 | Me | C₁₄H₂₉ | Br⁻ | 0.1 | 100 W |
| Example 15 | | | | 0.5 | 49 W |
| Example 5 | | | | 1 | 24 W |
| Example 16 | Et | C₁₄H₂₉ | Cl⁻ | 0.05 | 100 W |
| Example 17 | | | | 0.1 | 84 W |
| Example 18 | | | | 0.5 | 29 W |
| Example 7 | | | | 1 | 19 W |

As shown in Table 2, even when a microwave absorber made of a compound having a specified structure was used in an extremely small amount of 0.1 parts by weight, 0.05 parts by weight, or 0.01 parts by weight with respect to 100 parts by weight of resin, resin molding was possible within the range of output power (100 W or less in the present test example) that was allowable for the scale of resin pellet charge in microwave heating. Therefore, it was observed that the compound having the specified structure has high microwave absorption efficiency and is useful as an excellent microwave absorber in the heat-melting and molding of resins.

## Claims

1. A microwave absorber comprising an imidazolium salt represented by a formula (1): 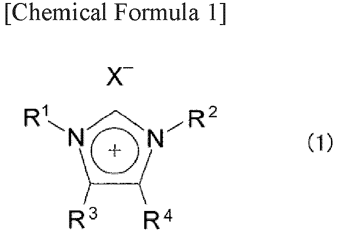 where R¹ represents a linear alkyl group having the number of carbon atoms equal to or less than the number of carbon atoms of an alkyl group having R², R² represents an alkyl group having 9 or more carbon atoms, R³ and R⁴ represent hydrogen or an alkyl group having 1 to 6 carbon atoms that may be same or different from each other, and X represents a halogen element.

2. The microwave absorber according to claim 1, wherein a number of carbon atoms of the alkyl group of R² is 9 to 22.

3. The microwave absorber according to claim 1, wherein the halogen element is chlorine or bromine.

4. The microwave absorber according to claim 1, wherein a difference between the number of carbon atoms of R¹ and the number of carbon atoms of R² is 10 to 12, and the halogen element is chlorine.

5. The microwave absorber according to claim 1, which is used by being mixed with a resin to be microwave-heated.

6. A resin composition comprising the microwave absorber according to claim 1 and a resin.

7. The resin composition according to claim 6, wherein the resin is a thermoplastic resin and has a pellet shape.

8. A method for heating a resin using the microwave absorber according to claim 1.

9. The method according to claim 8, wherein the microwave absorber is used at a ratio of 0.01 to 10 parts by weight to 100 parts by weight of the resin.

10. A method for producing a resin molded body, the method comprising:
a step 1 of heating the resin composition according to claim 6 with a microwave to provide a flowable resin composition; and
a step 2 of molding the flowable resin composition.

11. The method according to claim 10, further comprising a step of heating the flowable resin composition with a microwave to maintain flowability.

12. The method according to claim 10, wherein the step 2 is injection molding.
